Europäisches Patentamt

European Patent Office

Office européen des brevets

(11) Publication number: **0 160 467**
A2

(12)

# EUROPEAN PATENT APPLICATION

(21) Application number: **85302761.3**

(51) Int. Cl.⁴: **G 01 N 33/543**

(22) Date of filing: **19.04.85**

(30) Priority: **20.04.84 US 602297**

(43) Date of publication of application:
**06.11.85 Bulletin 85/45**

(84) Designated Contracting States:
**AT BE CH DE FR GB IT LI LU NL SE**

(71) Applicant: **SYNTEX (U.S.A.) INC.**
**3401 Hillview Avenue**
**Palo Alto, California 94304(US)**

(72) Inventor: **Litman, David Jay**
**1638 Oak Avenue**
**Los Altos California 94022(US)**

(72) Inventor: **Zuk, Robert Frank**
**837 Live Oak No. 3**
**Menlo Park California 94025(US)**

(72) Inventor: **Sizto, Ning Chung**
**332 Ely Place**
**Palo Alto California 94306(US)**

(74) Representative: **Armitage, Ian Michael et al,**
**MEWBURN ELLIS & CO. 2/3 Cursitor Street**
**London EC4A 1BQ(GB)**

(54) Enzyme immunoassay, bibulous support for use therein, and an assay kit.

(57) Methods and devices are provided involving heterogeneous enzyme assays. A simplified protocol for the assays results from incorporating enzyme substrate on a bibulous support to which is conjugated a member of a specific binding pair. Upon introduction of the support into the assay medium, the substrate diffuses into the medium, eliminating a separate development step for the assay.

EP 0 160 467 A2

Croydon Printing Company Ltd

-1-

## ENZYME IMMUNOASSAY, BIBULOUS SUPPORT FOR USE THEREIN, AND AN ASSAY KIT.

A number of methods have been developed for the rapid and accurate determination of analytes, such as haptens, antigens and receptors. One group of assays involves a bibulous support and an enzyme conjugate, where the presence or absence of a dye on the support is indicative of the amount of analyte in the assay medium. In developing these assays, it is desirable to simplify the protocol. Protocols which involve numerous independent manual steps, usually result in the introduction of numerous errors. Also, there can be greater variation in the results between different technicians. Furthermore, a multi-step protocol is usually tedious, unless the protocol can be automated. Automated assays normally require sophisticated machines, which would preclude the use of the assay outside of large clinical laboratories.

It is therefore of interest to be able to simplify presently existing assays by simplifying protocols, where reagents are provided in predetermined amounts and measurements by the user are avoided.

9555K                                        92130-FF

U.S. Patent No. 4,168,146 describes a test strip immunoassay. U.S. Patent No. 4,299,916 describes an enzyme assay employing a bibulous support and enzyme binding to the support in relation to the amount of analyte in an assay medium. See also U.S. Patent No. 4,391,904, a Continuation-in-part of 4,299,916. U.S. Patent No. 4,366,241 involves an alternative device for performing immunoassays. Application Serial No. 398,505, filed July 15, 1982, now U.S. Patent No. 4,435,504, describes an enzyme chromatographic immunoassay involving two enzymes.

An improved method for performing enzyme immunoassays is provided, where the enzyme immunoassay involves a specific binding pair member being linked to a bibulous support and the amount of enzyme which becomes bound to the support is related to the amount of analyte in a sample. The presence of the enzyme bound to the support results in color formation on the support as a result of an enzyme catalyzed reaction of a substrate leading to a colored product. The protocol is greatly simplified by impregnating at least a portion of the support with enzyme substrate, which substrate becomes available to the enzyme upon contact with the sample solution.

Enzyme immunoassays, involving the binding of an enzyme to a bibulous support through the intermediacy of a reaction between homologous members of specific binding pairs, are simplified and improved by impregnating the bibulous support with an enzyme substrate. The result of the assay is determined by a change in the development of color as a result of enzyme catalyzed product formation from the substrate. In addition to the presence of the impregnated substrate, a member of the specific binding pair will be non-diffusively bound to the bibulous support.

Various devices can be employed involving bibulous supports, where the assay medium results in a moving front. The devices can have the non-diffusively bound member of the specific binding pair located on only a small proportion of the total area which the solvent front travels or may extend over a major portion or all of the device which the solvent traverses. Similarly, the substrate may be impregnated on a portion of or on all of the area which the solvent traverses, usually only a portion. The area of binding of the member of a specific binding pair and impregnation of the substrate will be determined by the nature of the device.

One device which is of particular interest is described in U.S. Patent No. 4,168,146. This device, subject to modification, involves having a member of a specific binding pair being uniformly non-diffusively bound over a major portion of a bibulous strip. The end or tip of the device is immersed in a sample solution and the sample allowed to migrate upwards toward the other end of the device. After washing, the device is then immersed in a sample having a member of a specific binding pair bound to an enzyme, which binds to available sites on the device, which sites are available as a result of the presence or absence of the analyte. After washing the device, the device is then immersed in a developer solution having substrate for the enzyme, which results in a detectable color being present on the device in those areas where enzyme is present.

There have been numerous improvements made in the device, providing for simplified protocols. Of particular interest is the use of a combination of enzymes, where in addition to the member of the specific binding pair, an enzyme is also bound over a major portion of the device. A second enzyme is bound to the member of the specific binding pair, where the two enzymes are related in that the substrate of one is the product of the other. By employing this combination of enzymes, which may be referred to as "channeling," one can simplify the protocol by combining the analyte-enzyme conjugate with substrates for both the first and second enzymes. The result for the assay is determined by the farthest extent to which color develops, which may be predominantly a line or may extend over the entire area where the analyte-enzyme conjugate is bound. For further description of this assay, U.S. Patent Application Serial No. 398,505, now U.S. Patent No. 4,435,504, should be considered, which disclosure is incorporated herein by reference.

In a second device, a bibulous member is introduced into the assay sample. The bibulous member will be a small element mounted on a support, which element is completely immersed in the assay medium. Bound to the element will be a member of the specific binding pair and, as appropriate, an enzyme, which serves the same function as described above. An analyte-enzyme conjugate is employed which will bind to the element in proportion to the amount of analyte in the assay medium. One can remove the element from the assay medium, wash the element, and then immerse the element in a developer containing substrates for the enzyme which result in a colored insoluble product, so that the colored product is deposited upon the element in proportion to the amount of analyte-enzyme conjugate bound to the element. This device is further described in U.S. application serial no. 255,022 filed April 17, 1981, now U.S. Patent No. 4,391,904, which description is incorporated herein by reference.

In accordance with the subject invention, these devices or analogous devices are modified by impregnating into the bibulous element a substrate for the analyte-enzyme conjugate which results in production of a color. Under the conditions of the assay, the substrate is available to the enzyme and is rapidly transformed by enzyme catalysis to a colored product which binds to the bibulous element to provide a detectable signal, which can be related to the amount of analyte in the assay medium. By impregnating the bibulous element with the substrate providing the color development, the reagent mixture which is employed for preparing the assay medium is simplified, background can be diminished, and a more quantitative result achieved.

To further explain the subject invention, in the subsequent description of the subject invention, the following definitions will be used.

## DEFINITIONS

Analyte - The compound or composition to be measured, which may be a ligand, which is mono- or polyepitopic, antigenic or haptenic, a single or plurality of compounds which share at least one common epitopic site or a receptor.

Specific Binding Pair ("mip") - One of two different molecules, where one of the molecules has an area on the surface or in a cavity which specifically binds to a particular spatial and polar organization of the other molecule. The members of the specific binding pair are referred to as ligand and receptor ("anti-ligand"). For the most part, the receptor will be an antibody and the ligand will serve as an antigen or hapten and to that extent are members of an immunological pair. Therefore, each of the members may be referred to as a mip, it being understood that "mip" is intended to include all ligands and all receptors.

Ligand - Any organic compound for which a receptor naturally exists or can be prepared.

Receptor ("Anti-Ligand") - Any compound or composition capable of recognizing a particular spatial and polar organization of a molecule, i.e. epitopic or determinant site. Illustrative receptors include naturally occurring receptors, e.g. thyroxine binding globulin, antibodies, enzymes, FAB fragments, lectins and the like.

Label - The label may be any molecule conjugated to another molecule or support and where two molecules are involved, is arbitrarily chosen as to which molecule is the label. In the subject invention, the labels will be enzymes which are conjugated to the support and a mip.

Signal Producing System - The signal producing system has three or more components, at least one component being conjugated to a mip. The signal producing system produces a measurable signal which is detectable by external means, normally by measurement of the electromagnetic radiation, desirably by visual examination. The signal producing system involves enzyme substrates and chromophores, where chromophores include dyes which absorb light in the ultraviolet or visible region, phosphors, fluorescers and chemiluminescers.

Immunochromatograph - The immunochromatograph has a plurality of mips, either ligand or receptor, bound in a region to a bibulous support which allows for the movement of a liquid across the region with transport of the analyte and, as appropriate, any members of the signal producing system. The mips are non-diffusively bound to the support, either covalently or non-covalently. The area to which the mip is uniformly bound is referred to as the "immunosorbing zone." In addition, one or more members of the signal producing system is non-diffusively bound to the bibulous support, either covalently or non-covalently.

Materials

The enzymes which are employed will for the most part be those enzymes which provide a colored product from a product which does not absorb in the visible region, e.g. a leuco dye. The substrate will generally be one which is transformed by hydrolysis or a redox reaction. The product which results will be relatively insoluble in water and bind reasonably strongly to the bibulous support, so as to provide a detectable signal at or about the site where it is formed. Various enzymes which may be employed include such hydrolases as glycosidases, e.g. β-galactosidase, β-glucosidase, phosphatases, cholinesterase, etc., or other hydrolase. Among oxidoreductases, are such enzymes as peroxidases, oxidases, e.g. uricase, monoamine oxidase, glucose oxidase; dehydrogenases, e.g. glucose-6-phosphate dehydrogenase, or other peroxidases, oxidases or dehydrogenases.

A wide variety of dyes may be used which provide for the desired colored product. Particularly, many phenolic compounds can be capped, so that the capped product, which is unable to ionize, will be colorless. However, upon removal of the capping material, the product will be colored. Illustrative of such dyes are the fluoresceins, hydroxycoumarins, e.g. umbelliferone, etc. Various compounds which react with hydrogen peroxide, either enzymatically catalyzed or through the intermediacy of other compounds include naphthols, e.g. 4-chloronaphthol, ABTS, etc., that is, any compound which can be conveniently detected by visual or instrument observation. Therefore, the term dye intends a compound which allows for detection in a range which can be distinguished from the background. Usually this will be

9555K                                                          92130-FF

the visible range. The compound will generally absorb light in the region of about 350 - 650 nm, or be a compound which fluoresces, generally absorbing light below about 600 and emitting light above about 350, more usually above about 400 nm.

The member of a specific binding pair which is bound to the bibulous support will be the analyte, its homologous or reciprocal binding member, or analogs thereof. Therefore, the materials which are bound will be ligands, either haptenic or antigenic ligands, or receptors, either naturally occurring or synthetic.

The ligand analytes of this invention are characterized by being monoepitopic or polyepitopic, while the receptor analytes may have a single or plurality of binding sites. The polyepitopic analytes will normally be poly(amino acids), i.e. polypeptides and proteins, polysaccharides, nucleic acids, and combinations thereof. Such combinations or assemblages include bacteria, viruses, chromosomes, genes, mitochondria, nuclei, cell membranes and the like.

For the most part, the polyepitopic ligand analytes employed in the subject invention will have a molecular weight of at least about 5,000, more usually at least about 10,000. In the poly(amino acid) category, the poly (amino acids) of interest will generally be from about 5,000 to 5,000,000 molecular weight, more usually from about 20,000 to 1,000,000 molecular weight, and among hormones of interest, about 5,000 to 60,000 molecular weight.

An extensive listing of useful ligands may be found in U.S. Patent No. 4,275,149, the disclosure bridging columns 12 to 17, which disclosure is incorporated herein by reference.

The monoepitopic ligand analytes will generally be from about 100 to 2,000 molecular weight, more usually from about 125 to 1,000 molecular weight. The analytes of interest include drugs, metabolites, pesticides, pollutants, and the like.

A large number of analytes of interest are listed in U.S. Patent No. 4,275,149, columns 17 and 18, which disclosure is incorporated herein by reference.

For receptor analytes, the molecular weights will generally range from about $10^4$ to $2 \times 10^8$, usually from about $3 \times 10^4$ to $2 \times 10^8$, more usually from about $3 \times 10^4$ to $2 \times 10^6$. For immunoglobulins, IgA, IgD, IgE, IgG and IgM, the molecular weights will generally vary from about 160,000 to about $10^6$. Enzymes will normally vary from about 10,000 to 600,000 daltons. Natural receptors vary widely, being generally at least about 25,000 molecular weight and may be $10^6$ and higher, including such materials as avidin, thyroxine binding globulin, thyroxine binding prealbumin, transcortin, membrane surface proteins, etc.

Where a ligand is conjugated to another molecule or support, frequently the ligand will be modified to provide for a particular functional group at a particular site. This modification produces a product referred to as a ligand analog. U.S. Patent No. 4,275,149 also has an extensive description, bridging columns 18 and 19, of ligand analogs which description is incorporated herein by reference.

The device which is employed will either be an immunochromatograph or a dip-stick or other analogous device. The immunochromatograph will be described first.

Immunochromatograph

The immunochromatograph involves a bibulous support providing liquid travel through capillarity, a non-diffusively bound mip, and also includes one or more members of the signal producing system, that is, at least one substrate.

A wide variety of supports may be used of different dimensions, particularly thicknesses, different materials and different shapes. For the most part, the shape will be elongated, conveniently a rectangular strip. At least a portion of the strip will have a mip uniformly bound to the strip. The size of the strip will be governed to some degree by convenience in handling. Also, the immunosorbing zone must be of sufficient size to be able to accommodate all of the analyte which may be present in the concentration range of interest of the analyte. Where the protocol involves binding of both analyte and labeled mip, then the immunosorbing zone must include capacity for both the analyte and labeled mip.

A wide variety of bibulous supports may be used, which include both natural and synthetic polymeric materials, particular cellulosic materials, such as fiber containing papers, e.g. filter paper, chromatographic paper, etc., synthetic or modified natural occurring polymers, such as poly(vinyl chloride), cross-linked dextran, acrylates, nylon, polyvinyldifluoride, etc., either used by themselves or in conjunction with a ceramic material, such as silica.

The thickness of the immunochromatograph bibulous support will generally vary from about 0.05mm to about 2mm, more usually being about 0.1mm to 0.5mm, preferably from about 0.2mm to about 0.4mm. The structure of the paper may be varied widely and includes fine, medium fine, medium, medium coarse and coarse. The surface may be varied widely with varying combinations of smoothness and roughness combined with hardness and softness.

The immunochromatograph may be supported by a variety of inert supports, such as Mylar, polystyrene, polyethylene, or the like. The supports can be used as a backing spaced from the immunochromatograph, edging, or other structure to enhance the mechanical integrity of the immunochromatograph.

Dip-stick

The dip-stick will involve one or two elements, where one element is the sample element and the other element is the standard. The two elements are bibulous solid supports and will be referred to as the "support" or "solid support."

The bibulous solid support can be widely varied. Usually, the support will be chosen so as not to be strongly adsorbent for members of the signal producing system. Adsorption would deleteriously affect the assay and interfere with the measurement of the signal generated by the signal generating system. The support is also chosen so as to substantially retain its physical integrity during the assay. The support may take different forms, have different physical characteristics, can be of different chemical compositions and may be of one or more compositions, as a mixture of compositions or laminates or combinations thereof. The particular support will interact with the signal generating compound by desolubilization of the enzyme product onto the support.

The support may be of a variety of shapes and forms, as well as of varied dimensions depending on the manner of use and measurement. The support may be mounted on a base, such as a rod, tube or capillary, fiber, strip, disk, plate, cuvette, or the like. The support may be an integral part of the base or distinct from the base, as an applied layer having a relatively small thickness, usually at least 0.1μ, generally 10μ, or greater, depending on the nature of the support, ease of application and desired properties.

The support may be opaque, translucent or transparent. Preferably, the support will be penetrable

9555K                                                        92130-FF

by the signal generating compound to at least a depth of 0.1µ, more preferably at least 1µ, and particularly preferred at least 10µ.

The support may also be considered in accordance with its function. The support serves as a material which retains a discrete existence in the aqueous assay solution, so as to be discernable from the medium and usually separable from the medium. The support serves to support mips which are bound to it, so that they are incapable of diffusing through the solution independent of the support. In addition, the support acts as a support for the enzyme substrate and product, acting as a base for a deposited layer. The support is effectively non-fluid, discrete in that the support is distinguishable from the liquid medium in which the support is immersed, and provides a distinct base or foundation for supporting mips, members of the signal producing system or other compounds as appropriate, which are bound either covalently or non-covalently. The support may exist in a charged or non-charged form, being charged where such charge provides some advantage to the operation of the signal producing system.

Various materials may be employed, the primary considerations being the binding of the signal generating compound to the support, the absence of interference of signal generation, the ease of conjugating to the support, and the like.

A wide variety of organic and inorganic polymers, both natural and synthetic may be employed as the material for the bibulous support, which provide bibulous properties e.g. paper.

The particular dimensions of the support support will be a matter of convenience, depending upon the size of the samples involved, the protocol, the means for measuring the signal, and the like.

The support will usually be polyfunctional or be capable of being polyfunctionalized, so as to allow for covalent bonding between the mip and the support, as well as the other compounds which must be conjugated. Functional groups which may be present on the support and used for linking can include carboxylic acids, aldehydes, amino groups, cyano groups, ethylenic groups, hydroxyl groups, mercapto groups and the like. The manner of linking a wide variety of compounds to the various supports is well known and is amply illustrated in the literature. See for example "Immobilized Enzymes", Ichiro Chibata, Halsted Press, New York, 1978, and Cuatrecasas, J. Biol. Chem. 245 3059 (1970).

The length of the linking group may vary widely depending upon the nature of the compound being linked, the effect of the distance between the linked compound and the support on the linked compound's properties, the potential for cross-linking of the linked compound, and the like.

The linking group may be a bond or have up to about 12, usually not more than about 10 atoms in a chain. The linking group may be aliphatic, alicyclic, aromatic, heterocyclic, or combinations thereof. The total number of atoms of the linking group will be not more than about 20, usually not more than about 16 atoms other than hydrogen, which will be carbon; oxygen as oxy or oxo, both oxo-carbonyl and non-oxo-carbonyl; nitrogen as amino or amido, and sulfur as thio or thiono. Illustrative groups include methylenecarbonyl, succinimidyl, α-haloacetyl, thiomethylene, glycyl or polyglycyl, succindioyl, maledioyl, glutardialkylidene, methylenephenyldiazo, and ureido.

For both devices, a mip will always be bound, covalently or non-covalently to the support. Depending upon the nature of the protocol, the amount of bound mip may be limited or in excess of the highest amount of analyte which can be expected to be found in the sample based on binding sites of the analyte. In addition, the signal producing system may also be bound to the solid support. Frequently, the amount of the member of the signal producing system which is conjugated will be rate limiting, and thus, large excesses will be employed.

In preparing the bibulous support, the bibulous support may be activated with reactive functionalities to provide for covalent bonding of the organic materials to be conjugated to the support. These materials are other than the enzyme substrate. Various techniques may be used to activate the bibulous support, including functionalization with an acyl group e.g. carbonyldiimidazole, treatment with cyanogen bromide or difunctional agents such as glutaraldehyde, succinic acid, etc. Methods for binding of a wide variety of materials to a bibulous surface may be found in the literature. See for example, U.S. Patent No. 4,168,146.

The amount of mip which is bound to the support will vary depending upon the size of the support and the amount required to bind all of the analyte and, as required, labeled mip. Generally, the amount of mip will range from about $10^{-5}$ to $10^{-14}$ moles/cm$^2$, more usually from about $10^{-7}$ to $10^{-12}$ moles/cm$^2$. The number of moles per unit area will be varied in order to insure that there is sufficient discrimination in the concentration range of interest for the distance traversed by the analyte.

Both the specific binding pair member and the enzyme, as appropriate, may be bound to a variety of supports by adsorption, rather than covalent bonding. This will involve contacting the bibulous support with a solution containing the specific binding pair member and enzyme, removing the bibulous support from the solution and allowing the bibulous support to dry. Alternatively, the solution containing the specific binding pair member and enzyme may be applied by spraying, painting, or other technique which will provide uniformity.

Usually, large sheets will be used which may then be cut to the appropriate dimensions.

The bibulous support to which is bound the specific binding pair member and, as appropriate, the enzyme, will usually be washed prior to the binding of the substrate. Binding of the substrate will normally involve contacting the bibulous member with the substrate in conjunction with other additives generally in an aqueous medium. Alternatively, depending upon the dye, the bibulous support may be impregnated concomitantly with the dye when binding the specific binding pair member to the bibulous support. Alternatively, the enzyme, when present, may be combined with the substrate and bound to the bibulous support to which the specific binding pair member has been previously bound. Thus, either one or two steps will be involved, usually two steps.

The substrate-containing solution which is applied to the bibulous support will suitably be a polar solution, e.g. aqueous, alkanal, alkanone, etc., particularly low molecular inert (1-4 carbon atoms) organic polar solvents such as acetone. Other additives which may be included in the solution are poly(vinyl alcohol), hydroxylamine, buffers, detergents, etc. The hydroxylamine will generally range in an amount from about 10 to 100 mM, while the poly(vinyl alcohol) will generally range in an amount from 0.1 to 1 weight percent. Depending upon the nature of the dye, the amount of dye in the medium will vary, generally ranging in concentration from about 0.1-2, more usually from about 0.2-1, mg/ml. Various buffers may be employed, such as phosphate, tris, carbonate, etc. The pH will be related to the nature of the substrate, generally ranging from about 6-9.

After sufficient time for the bibulous element to have uniformly absorbed the substrate containing solution, the bibulous element may be treated in a variety of ways. The bibulous element may be blotted dry and then dried at ambient or elevated temperatures, generally below about 65°C, frozen, and lyophilized, or the like. The significant factor is that any treatment does not interfere with the activity of the various materials bound to the support, nor affect their relative distribution.

The bibulous support may be coated with a wide variety of materials to provide for enhanced properties. Coatings may include protein coatings, polysaccharide coatings, sugars or the like, which are used particularly to enhance the stability of the materials conjugated to the support. In addition, these compounds may provide for improved binding of the materials bound to the support.

Methods for carrying out assays with the two devices will now be considered. The immunochromograph involves a bibulous support which provides a stationary solid phase and an assay medium which provides a moving liquid phase. The region in which the mip is uniformly non-diffusively bound to the bibulous support is referred to as the immunoabsorbing zone. The analyte from the sample will traverse this zone being carried along with a solvent whose front traverses the zone. The analyte, which is the homologous or reciprocal mip to the mip bound to the support, becomes bound to the support through the intermediacy of mip complex formation. The signal producing system provides the manner in which the area in the immunosorbing zone to which the analyte is bound may be distinguished from the area in which it is absent, so that the distance from a predetermined point on the immunochromatograph is a measure of the amount of analyte in the sample.

The incremental movement of the sample through the immunosorbing zone results from dissolving the sample in an appropriate solvent and the transport of the solution through the immunosorbing zone due to capillarity.

The solvent will normally be an aqueous medium, which may be up to about 40 weight percent of other polar solvents, particularly oxygenated solvents of from 1 to 6, more usually of from 1 to 4 carbon atoms, including alcohols, ethers and the like. Usually, the cosolvents will be present in less than about 20 weight percent.

The pH for the medium will usually be in the range of 4 - 11, more usually 5 - 10, and preferably in the range of about 6.5 - 9.5. The pH is chosen to maintain a significant level of binding affinity of the mips. Various buffers may be used to achieve the desired pH and maintain the pH during the elution. Illustrative buffers include borate, phosphate, carbonate, tris, barbital and the like. The particular buffer employed is not critical to this invention, but in individual assays, one buffer may be preferred over another.

Desirably, from about 0.05 to 0.5 wt. % of a non-ionic detergent is included with the sample. Various polyoxyalkylene compounds may be employed of from about 200 to 20,000 daltons.

Moderate, and desirably substantially constant, temperatures are normally employed for carrying out the assay. The temperatures for the elution and production of a detectable signal will generally be in the range of about 2°-50°C, more usually in the range of about 15°-50°C, and frequently will be ambient temperatures, that is, about 15°-25°C.

The concentration of analyte which may be assayed will generally vary from about $10^{-4}$ to about $10^{-15}$ M, more usually from about $10^{-6}$ to $10^{-14}$ M. Considerations, such as whether the assay is qualitative, semi-qualitative, or quantitative, the particular detection technique; the concentration of the analyte of interest; and the protocol will normally determine the concentration of the other reagents.

In carrying out the assay, the protocol will normally involve dissolving the sample into the eluting solvent. The sample may be derived from a wide variety of sources, such as physiologic fluids, illustrated by blood, serum, plasma, urine, ocular lens fluid, spinal fluid, etc., chemical processing streams, food, pesticides, pollutants, etc.

One end of the chromatograph will then be contacted with the diluted sample containing solvent, which will normally be a buffered aqueous medium which may contain one or more members of the signal producing system. Where a member of the signal producing system is present, at least one member will be conjugated to a mip to provide a mip-label conjugate.

Sufficient time will be allowed for the solvent front to completely traverse the immunosorbing zone. The zone has a sufficient amount of mip to insure that all of the analyte becomes bound in said zone without exhausting the mip bound in the zone.

While various protocols may be employed, for the most part, the assay solution will involve the sample, the analyte, and the specific binding pair member-conjugate, as well as any additional reagents, such as buffer, detergents, additional substrates with the enzyme, etc. Various non-ionic detergents may be employed, generally being present in from about 0.01-5% weight percent of the assay medium. Where other substrates are present, there will be present an excess amount to insure that they are not rate limiting. The particular concentration which provides a useful result may be determined empirically.

The assay medium will be allowed to traverse the immunosorbing zone for sufficient time, so that the solvent front has traversed all or substantially all of the immunosorbing zone. The resulting immunochromatograph will then be allowed to stand for sufficient time for color to develop and the height of the color may be read as indicative of the amount of analyte in the assay medium.

For the dip-stick, the dip-stick will be immersed in the assay medium, which will have similar components to that indicated above, having at least the enzyme conjugate and the sample. After sufficient time for the reaction of the enzyme conjugate with its reciprocal binding member bound to the bibulous element, the element may be removed from the assay medium and either read directly or sufficient time permitted for additional product to be formed to enhance the observable signal. Washing may or may not be necessary.

As a matter of convenience, the device may be provided with the other reagents for use in the assay. The other reagents will at least include the enzyme conjugate and as appropriate, buffers, additional substrates, stabilizers, or the like, where the amounts of the various active ingredients are provided to optimize the sensitivity of the assay.

<u>EXAMPLES</u>

The following examples are offered by way of illustration and not by way of limitation.

## EXAMPLE 1

### A. Paper Activation

A roll of Whatman 31ET (0.53mm thickness, flow rate 225 mm per 30 min with water) chromatography paper (20" x 25') was rolled with nylon screen (35 mesh) into a cartridge and inserted into a cylindrical reactor. The paper was activated by circulating 4.5 l. of 0.2M carbonyldiimidazole/$CH_2Cl_2$ solution through the reactor for 90 minutes using an Eastern centrifugal pump (3400 RPM, 4.5 l./min). The paper was then washed 4 times with 4.5 l. $CH_2Cl_2$ and desolvated by passing dry $N_2$ gas through the reactor for 150 minutes.

### B. Protein Immobilization

Whatman 31ET chromatography paper was immobilized with protein as follows: A sheet of activated paper (230mm x 180mm) was immersed in 75 ml of a solution containing 10 µg/ml of glucose oxidase-amine (prepared as described in U.S. Patent No. 4,435,504, Example 3) and 2.0 mg/ml antibody to theophylline (anti-theophylline) in 0.1M, pH 7.0, phosphate buffer, and incubated at ambient temperature for 3.5 hours. The paper was then washed with 8 l. of 0.1M phosphate buffer over 45 minutes, followed by washing with 4 l. of distilled water over 15 minutes. The paper was then immersed in a solution containing 0.5% (W/V) polyvinyl alcohol (20/30) and 400 µg/ml of 4-chloronaphthol (4-Cl-1-naphthol) and incubated for 20 minutes, followed by blotting. The paper was dried in a suction tunnel dryer for 6.5 minutes at 65°C.

### C. Assay Protocol

The immobilized paper was slit into 90 x 6 mm strips. Strips were dipped into 0.5 ml wicking developer which consisted of a 2% theophylline solution (ranging from 0 to 40 µg/ml), 75 ng/ml horseradish peroxidase (HRP)-theophylline conjugate (prepared as described in U.S. Patent No. 4,435,504, Example 2), 200 µg/ml 4-Cl-1-naphthol, 50 mM glucose, and 1 mg/ml bovine serum albumin (BSA) in 0.1M, pH7.0 phosphate buffer. Color developed as the solution wicked up. The assay strip was then removed and the height of the color front was measured (from the bottom of the strip). The total assay time was 10-15 minutes.

D.   Results

The above protocol was followed and the results are shown in the following table:

| Theophylline* (µg/ml) | Distance of front from bottom of strip (mm) |
|---|---|
| 0 | 9.5 |
| 2.5 | 13.5 |
| 5.0 | 19.3 |
| 10.0 | 23.5 |
| 20.0 | 29.5 |
| 40.0 | 47.8 |

*2% solution

EXAMPLE 2

A conjugate of horseradish peroxidase and ampicillin was prepared as follows. To 2 ml of 8.24 mg/ml horseradish peroxidase in water was added 0.5 ml of 0.0124 M sodium periodate, and the mixture was stirred for one hour. The mixture was subjected to chromatography on a Sephadex® PD-10 column, eluting with 5 mM acetate buffer, pH 4.5. The protein fractions were pooled to give 3.5 mls. To the pooled oxidized HRP fractions was added, 0.5 ml of 2 M phosphate buffer, pH7.0, containing 38.5 mg ampicillin. The reaction was stirred for 30 minutes at room temperature; then 0.65 ml of a 10 mg/ml sodium cyanoborohydride solution was added with stirring. The reaction mixture was incubated overnight at 4° and was then dialyzed extensively against 0.1 M phosphate, 0.2 M sodium chloride, pH 7.0.

Whatman 1C (thicknes 0.16 mm, flow rate 130 mm per 30 min with water) chromatography paper was activated employing carbonyldiimidazole in a manner as described in Example 1. The activated paper (12.5 cm$^2$) was immersed in a solution of 100 μg/ml antibody to horseradish peroxidase (IgG), 1 mg/ml glucose oxidase-amine, 0.1 M phosphate, 0.2 M NaCl, pH7. The paper was then dried and impregnated by immersing in an acetone solution of 4-Cl-1-naphthol (impregnating solution) at varying concentrations (1, 5, 10, and 50 mg/ml) for sufficient time to insure complete wetting. The paper was then blotted and air dried.

Strips prepared from the above paper were then immersed in an assay solution (50 mM glucose, 300 ng/ml horseradish peroxidase-ampicillin conjugate (the ampicillin played no role in the assays), 2 mg/ml BSA, 0.1 M phosphate, 0.2 M NaCl, pH7) for 10 min and the reflectance from the strip resulting from the oxidized 4-Cl-1-naphthol was determined.

The following table indicates the results.

| 4-Cl-1-naphthol (mg/ml)* | Reflectance (avg. 3 results) | % Reflectance of Control |
|---|---|---|
| 0 (Control)** | 16.2 | - |
| 1 | 8.2 | 50. |
| 5 | 17.7 | 109. |
| 10 | 18.3 | 112. |
| 50 | 16.3 | 100. |

\* concentration of 4-Cl-1-naphthol in impregnating solution
\*\* contained 200 μg/ml 4-Cl-1-naphthol.

At concentrations of the naphthol dye in the impregnating solution of 5 mg/ml or above, the results with the strips impregnated with 4-Cl-1-naphthol are equivalent or better to those results obtained wherein

the 4-Cl-1-naphthol is in solution at a level commonly used in the developer solution for the known assay.

It is evident from the above results that protocols can be simplified by having the enzyme substrate impregnated into a bibulous support for use in enzyme immunoassays. Thus, the result can be directly achieved without having to transfer the bibulous support from the assay medium containing the sample to a developer solution. This system is particularly effective where two enzymes are used, instead of one, and can be used where one employs either a migrating liquid medium or a stationary liquid medium.

CLAIMS:

1. An enzyme immunoassay having a member of a specific binding pair bound to a bibulous support and a conjugate of an enzyme with a member of said specific binding pair, where a substrate for said enzyme undergoes enzyme catalysis to produce a substantially water insoluble detectable product,

characterised by:

impregnating said bibulous support with a sufficient amount of said substrate to provide for a detectable signal over the analyte concentration range of interest.

2. A method according to Claim 1, wherein said bibulous support is an elongate strip and said assay medium provides a mobile liquid phase traversing said strip.

3. A method according to Claim 2, having a second enzyme bound to said strip, where said second enzyme and said enzyme of said conjugate are related by the substrate of one being the product of the other.

4. A method according to Claim 1, wherein said bibulous support is a small element which is completely immersed in said assay medium.

5. A method according to Claim 4, wherein a second enzyme is bound to said bibulous support, wherein said second enzyme is related to said enzyme conjugate by the product of one being a substrate of the other.

9555K

92130-FF

0160467

6.    A bibulous support for use in a method according to Claim 1 having a member of a specific binding pair non-diffusively bound thereto and an enzyme substrate.

7.    A bibulous support according to Claim 6, wherein said support is an elongate strip.

8.    A bibulous support according to Claim 6, wherein said bibulous support is a small element for complete immersion in an assay medium.

9.    A kit comprising in a packaged combination a bibulous support according to Claim 6, and an enzyme conjugate wherein said enzyme conjugate catalyzes the transformation of said substrate to a colored product.

10.    A kit according to Claim 9, wherein said enzyme conjugate is combined with buffer and at least one stabilizer.